# EUROPEAN PATENT SPECIFICATION

(11) **EP 2 139 371 B1**
(45) Date of publication and mention of the grant of the patent: **02.10.2013**
(21) Application number: 07735097.3
(22) Date of filing: 13.03.2007
(51) Int. Cl.: A47L 11/34, B08B 3/00, A61L 2/07

(54) **STEAM CLEANER AND METHOD FOR STEAM CLEANING**
DAMPFREINIGER UND DAMPFREINIGUNGSVERFAHREN
APPAREIL DE NETTOYAGE À LA VAPEUR ET PROCÉDÉ DE NETTOYAGE À LA VAPEUR

(43) Date of publication of application: 06.01.2010
(73) Proprietor: Proventec plc, London EC2A 4ES (GB)
(72) Inventor: TEERLINK, Peter, NL-3065 NG Rotterdam (NL)
(74) Representative: Vaughan, Christopher Tammo
(86) International application number: PCT/IB2007/050846
(87) International publication number: WO 2008/110875

(56) References cited:
- EP-A- 0 634 177
- EP-A- 0 928 595
- US-B1- 7 051 462

## Description

The invention relates to a steam cleaner. Steam cleaners are known and for example marketed as DeepClean® by Osprey Deepclean Nederland BV, Capelle aan de IJssel.

Steam cleaners can be used for cleaning artefacts such as products, surfaces of floors, walls or ceilings, equipment and the like. To clean these artefacts different utensils can be used, which can be coupled to a steam hose connected to or part of said steam cleaner. Preferably movable steam cleaners are used, such as portable steam cleaners or steam cleaners on wheels.

From EP-A-0928595 a steam cleaner is known, having a water tank, steam forming means and a box for storing cleaning utensils.

An object of the present invention is to provide a steam cleaner which can even better clean artefacts or which can be used for an even greater variety of artefacts, surfaces and other cleanable objects.

A steam cleaner of the present invention is set out in claim 1.

It has been recognised in the present application that although steam cleaners can in general clean artefacts such as products, surfaces and the like accurately, the utensils used with such steam cleaners are a source for bacteria and other contaminations. They are used on different artefacts and bacteria and other contaminations will gather in and on said utensils during use, which can be transferred from one artefact to another. They can be cleaned in between, for example by rinsing or brushing, but this is cumbersome and does not always lead to clean utensils.

In the present invention a tank or box is provided with the steam cleaner, in which the utensils can be cleaned using steam generated with said steam cleaner. The utensils can be thoroughly cleaned at hand and can even be sterilized on site, resulting in a seduction of contamination and preventing transfer of contamination.

It should be recognised that the tank or box can be part of the steam cleaner, for example the receiving tank for waste, but can also be a separate tank or box, which can be attached to or carried with a steam cleaner.

In a further aspect a steam cleaner can be characterised by at least one steam hose is provided, having a first end for cooperation with the at least one socket and an opposite second end provided with a coupler for coupling to the at least one inlet.

The steam hose can be connected to the inlet for steam into the receiving tank of tool box, for cleaning and preferably sterilizing said utensils inside said box or tank. Alternatively a (semi) permanent connection can be provided between said steam generating means such as the heating means and the tank or box, provided with a steam regulator in said connection for controlling the inlet of steam.

In a still further aspect the present invention can be characterised by a method for cleaning for cleaning artefacts, wherein at least one artefact is cleaned using a steam cleaner with at least one cleaning utensil, wherein after cleaning at least part of said artefact said at least one cleaning utensil is positioned in a tool box or receiving tank, wherein steam generated with said steam cleaner is introduced into said tool box or receiving tank.

In a still further aspect the invention relates to a method for cleaning artefacts using a steam cleaner, wherein said artefact is positioned within a receiving tank or tool box, after which steam is generated with said steam cleaner and is introduced into said tank or box for cleaning and preferably sterilising said artefact.

In the sub claims further advantageous embodiments are disclosed.

For a better understanding of the invention different embodiments will be described hereafter, as examples and in no way limiting the invention, with reference to the drawing. Therein:
fig. 1A shows schematically in frontal view part of a steam cleaner;
fig. 1B shows schematically in side view a steam cleaner, with a side partially broken away along the line IB - IB in fig, 1C;
fig. 1C shows a steam cleaner according to fig. 1A and B, in rear view;
fig. 2 shows, partially in cross section, in side view schematically a receiving tank of a steam cleaner, with part of a steam hose having two channels;
fig. 3A shows schematically in side view a second embodiment of a steam cleaner, with a side partially broken away along the line IIIA - IIIA in fig. 3B;
fig. 3B shows a steam cleaner according to fig. 3A, in rear view;
fig, 4 shows, partially in cross section, in side view schematically a tool box for a steam cleaner, with part of a steam hose;
fig. 5 shows in perspective view a receiving tank or tool box;
fig. 6 shows part of a steam cleaner, connecting the receiving tank or tool box and the steam generating means; and
fig. 7 shows a detail of an inlet of a tank or box according to fig. 5.

The embodiments shown and described hereafter are only presented as examples of the invention and should by no means be considered limiting the scope of the present invention. Many variants are possible within the scope of the present invention. In the embodiments shown identical or similar parts have the same or similar reference signs. Combinations of parts of the embodiments shown are also considered to have been disclosed.

In this application artefact has to be understood as encompassing but not limited to products, machines, tools, utensils, constructions and other material objects at least partly made by man, including but not limited to furniture such as tables, chairs, cup boards and beds, mattresses and cushions, carpentry, window decoration, utensils such as cutlery and tools, flooring such as floor boards, carpets and plastic flooring, ceilings and walls and other materials.

In this application steam has to be understood as including but not limited to dry and wet steam, overheated steam, steam including additives such as cleaning agents, disinfectants, anti bacterial agents, odorants and the like, although steam made of clear water is preferred, which steam is preferably at a pressure and temperature for sterilizing.

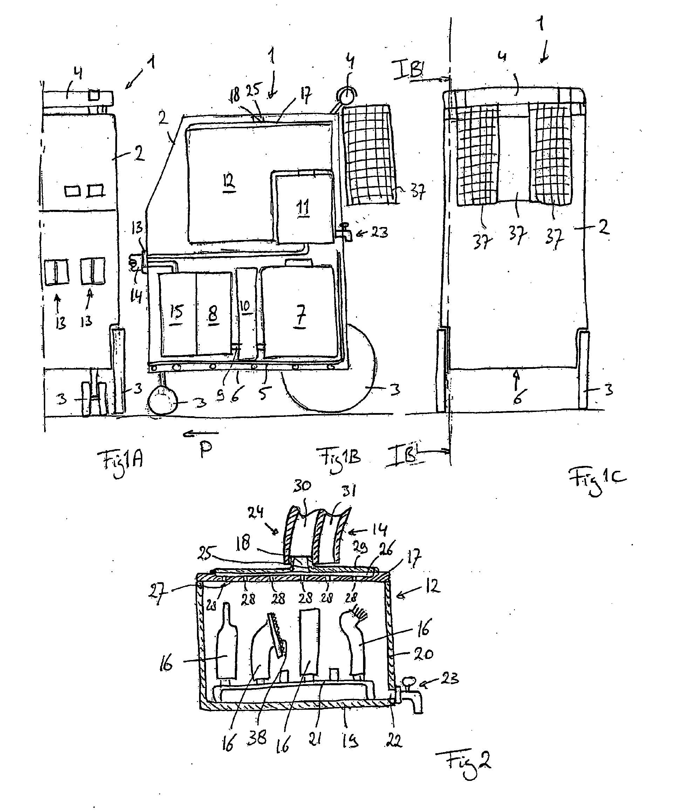

In fig. 1A - C in respectively front, side and rear view a steam cleaner 1 is shown, in fig. 1B partially in cross section, comprising a housing 2 carried on wheels 3, such that it can be rolled around. A pushing bar 4 may be provided for pushing and/or pulling of the steam cleaner 1 around. Within the housing 2 a drawer like sliding element 5 is provided near a bottom 6, in which sliding element 5 a water tank 7 and heating means 8 are provided, in fluid connection by a connector 9 in a known manner, suitable for inserting water from the water tank 7 into the heating means 8 for heating said water, such that it can be transferred into steam. An electronic control box 10 is furthermore provided on said sliding element 5, for controlling inter alia the heating means 8. Such steam generating means are generally known and for example used in the referenced Deepclean® steam cleaner, incorporated herein by reference for the at least the steam generating means. By placing these elements 7, 8, 9, 10 in the sliding element they can easily be slid out of said housing in the direction P for maintenance, for example cleaning or repairs and/or for filling the water tank 7. The elements and further electronically controlled elements in the steam cleaner 1 are preferably interconnected by connectors that can easily and readily be unplugged and re-plugged, such that each component can be exchanged easily, should such be necessary, for example when a failure occurs, such that the down time of the steam cleaner can be minimised or even avoided.

Within the housing furthermore suction means 11 and a receiving tank 12 are provided. Furthermore at least one connecting socket 13 for connecting a steam hose 14 is provided, which is or can be brought into fluid connection with both the suction means 11 and a steam tank 15 provided within housing 2, in fluid connection with the heating means 8, such that steam can be pressurized in said steam tank 12 and can be expelled through said socket 13 and said steam hose 14, for cleaning an artefact. By the suction means 11 dirt collected from the artefact can be sucked into the receiving tank 12, together with at least part of the steam which will have condensed or otherwise liquefied on or near said artefact.

Tests have shown that artefacts as indicated before and others can be thoroughly cleaned using steam, and can even be sterilized. However, it has been proven that tools or utensils 16 used with such steam cleaner 1 can for a source for infection or transfer of dirt, bacteria and other undesirables, since these themselves are not sufficiently cleaned. Especially in environments where cleanliness is essential, such as clean rooms, hospitals and other such areas, but also in kitchens, toilets and the like, therefore it is essential that clean cleaning utensils 16 are used.

In the steam cleaner 1 the receiving tank 12 is provided with a lid 17 and at least one inlet 18 for steam into said receiving tank 12. This inlet 18 may also be referred to as steam inlet 18. In fig. 2, 5 and 7 such receiving tank 12 is shown in more detail, in partial cross section. The tank 12 comprises a bottom 19, a wall 20 and a lid 17, wherein said inlet 18 is provided in said lid, although this could also be provided elsewhere, for example in said wall, preferably close to said lid or at least close to an upper part of said tank 12. A rack 21 is provided which can fit inside said tank 12, preferably resting on said bottom 19 or made integral with said tank 12, although preferably said rack can be removed from said tank 12. An outlet opening 22 is provided, preferably near said bottom 19, for draining said receiving tank 12, preferably provided with a closure 23 such as a tap. The receiving tank 12 can thus be drained from waste such as any liquid material such as water mixed with dirt as received from the suction means 11. On the rack 21 cleaning utensils 16 can be placed, such ass but not limited to utensils 16 as used with the steam cleaner 1, for example suitable for attaching to an end 24 of the steam hose 14 for cleaning artefacts. Also smaller artefacts could be placed on said rack 21. The rack together with the utensils 16 and/or artefacts can then be placed inside said receiving tank 12 (once drained), after which the lid 17 can be closed and steam can be generated and introduced onto said tank 12 through said inlet 18, for cleaning and preferably sterilising said utensils and/or said artefacts inside said receiving tank 12. Preferably the tank 12 can be hermetically sealed during such cleaning and/or sterilising. Then the utensils and/or artefacts can be removed and reused. Thus in an easy manner sterile utensils 16 such as cleaning tools and artefacts can be obtained and the risk of transfer is minimized. A tool box described hereafter can be of the same or similar construction.

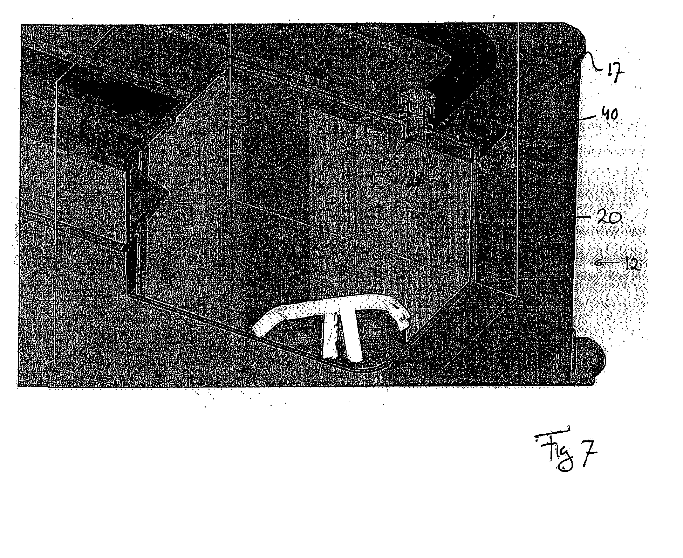

As is shown in fig, 2 the end 24 of the steam hose 14 can be connected to a connector 25 on the lid 17, such that steam can easily be generated by the steam cleaner and be introduced into said tank 12 through said inlet 18. As is shown in fig. 2 and 7 the inlet 18 can comprise a slot 26 in the lid, having a bottom 27 in which series of openings 28 is provided, A cover, shown as a closing strip 29 is provided over said slot 26, closing it off. The connector 25, which can be closable by a cap 40, can be attached to the strip 29. The slot 26 shown can have a substantially U-shaped configuration, for distributing the steam more evenly through the tank 12 or tool box 33 as will be described later, which can have a similar configuration. A lifting means such has a hanger 41 can be provided for easy lifting of the tank 12 or box 33.

In a steam cleaner 1 according to fig. 1 and 2 the steam hose 14 has at least two channels 30, 31. A first channel 30 is provided for leading steam to and/or through a utensil or tool 16, the other channel 31 is provided for connecting to the suction means 11, for sucking up waste, dirt water and the like and dumping this into the receiving tank 12. In a steam cleaner 1 two sockets 13 can be provided, for attaching two steam hoses 14, such that no utensils 16 have to be changed or at least less frequently. More or only one such socket 13 could be provided.

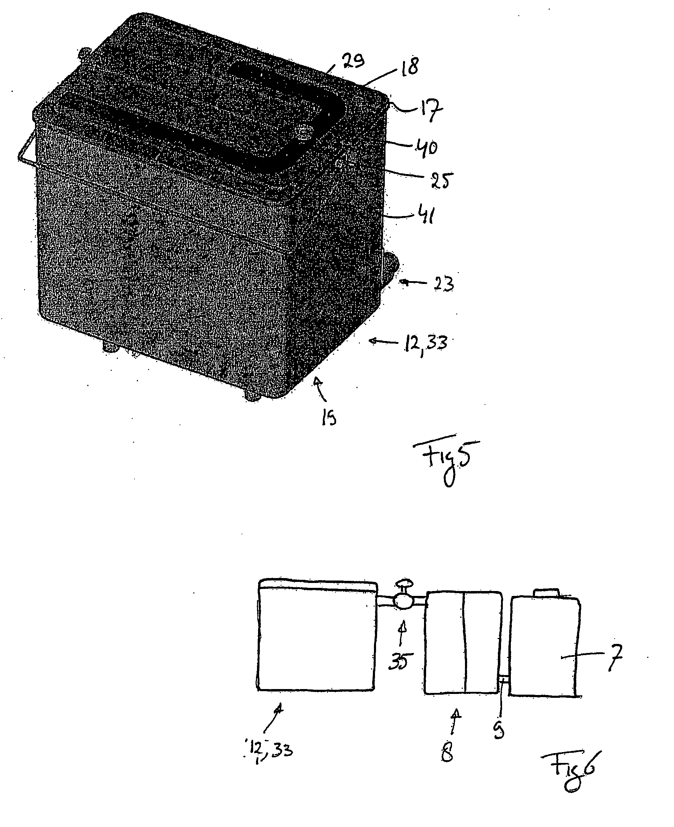

In fig. 6 part of an embodiment is shown in which the receiving tank 12 and the steam generating means 7, 8, 10 are connected directly, wherein a valve 35, preferably electrically operable, is provided for letting steam into the receiving tank 12 or tool box 33. A tool box may also be partly or entirely flexible.

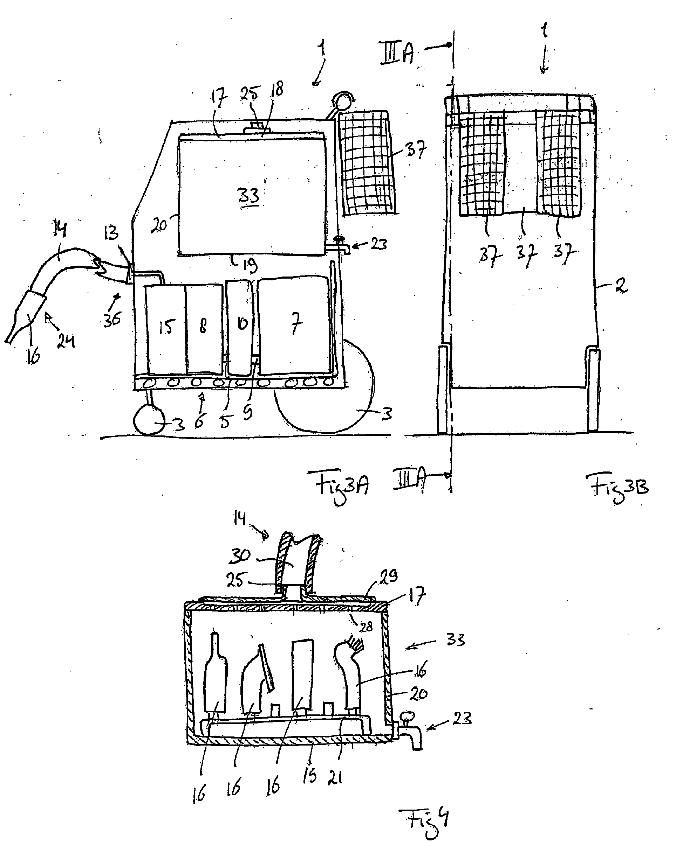

In fig. 3A and B a steam cleaner 1 is shown comparable to the one according to fig. 1, but having no suction means. In this embodiment a steam hose 14 can be inserted into or is connected to the or a socket 18 and can have only the first channel 30. In this embodiment a tool box 33 is provided, comparable to a receiving tank 12 as shown and described above, in which the same or similar reference signs have been used for similar part. This tool box 33 may have means 22 for draining. A rack 21 is or can be provided inside the tool box 33, for carrying utensils and/or relatively small artefacts, as described with reference to the receiving tank 12. In this embodiment the tool box can be integrated in the steam .cleaner 1 or can be provided separate, such that it can for example be placed on top of the housing 2 or next to the steam cleaner. It may also be used with different and/or various steam cleaners.

In fig. 4 a tool box 33 is shown, partially in cross section, in which an end 24 of the steam hose 14 is connected to the inlet 18 of a tool box 33, which steam hose 14 is connected to the steam generating means with the opposite end 36. Steam generated with the steam cleaner 1 can be inserted into the tool box 33 for cleaning and/or sterilising the utensils and/or artefacts.

A steam cleaner 1 is for example suitable for cleaning a series of surfaces of furniture, such as mattresses or cushions, wherein utensils such as steam cleaning tools 16 can be cleaned or even sterilised in situ, such that transfer of contamination such as bacteria is prevented. In the same manner various other artefacts can be cleaned in series.

In fig. 1 and 2 a number of receptacles 37 is provided, attached to the housing 2 or the bar 4, for receiving utensils and/or tools 16 and/or cloth 38 such as micro fibre cloth for use with the steam cleaner 1, which may also be cleaned in the receiving tank 12 or tool box 33. The receptacles may be detachable and for example foldable.

The present invention is by no means limited to the embodiments shown and described. Many variations are possible within the scope of the invention as claimed. For example, the receiving tank 12 and/or tool box 33 may be designed differently, for different tools or utensils. Moreover, they may be designed such that a tool or utensil 16 attached to a hose 14 can be inserted for cleaning and/or sterilisation. Different lids 17 and/or tanks 12 and/or boxes 33 may be provided, for example rigid or flexible, and they could be coupled directly or indirectly to the hose 14. Moreover, a disposable or reusable container such as a cup or bag could be provided for attachment to the end of the hose, in which artefacts, tools and/or utensils could be cleaned and preferably sterilised and possibly stored for later use. Inlets 18 may be designed differently and may be exchangeable, for example such that for different tools, utensils and/or artefacts different inlets can be provided, each defining a proper distribution of steam inside said tank 12 or box 33. A steam cleaner may be portable, for example provided with straps or a frame for carrying such or may be stationary. The rack 21 could be dispensed with, the tools being inserted directly into said box 33 or tank 12. The housing could be open at for example a top side for approach of the tank 12 or box 33, but could also be provided with a lid or other cover for protection.

## Claims

1. Steam cleaner (1), comprising a water tank (7), heating means (8) for forming steam and at least one connecting socket (13) for a steam hose (14), wherein furthermore a tank or box (12, 33) is provided, **characterized in that** the tank or box is provided with at least one inlet (18) for steam into said tank or box (12, 33), a receiving tank (12) for waste and a suction device (11) for sucking waste into said receiving tank (12).

2. Steam cleaner (1) according to claim 1, wherein said tank or box comprises a tool box (33).

3. Steam cleaner (1) according to claim 1 or 2, wherein said tank or box (12, 33)is provided with a lid (17), wherein said at least one inlet (18) is provided in or near said lid (17).

4. Steam cleaner (1) according any one of the previous claims, wherein the tank or box (12, 33) is provided with a series of inlets (18).

5. Steam cleaner (1) according to any one of the previous claims, wherein at least one steam hose (14) is provided, having a first end for cooperation with the at least one socket (13) and an opposite second end provided with a coupler for coupling to the at least one inlet (18).

6. Steam cleaner (1) according to any one of the previous claims, wherein the tank (12) or box (33) is provided with an outlet opening (22), preferably provided with a closure (23).

7. Steam cleaner (1) according to any one of the previous claims, wherein cleaning utensils (16) are provided for connecting to said at least one socket (13), preferably via a steam hose (14), or for use with said steam cleaner (1) separately, wherein at least one of said utensils (16) can be placed inside said tank or box (12, 33).

8. Steam cleaner (1) according to claim 7, wherein a rack (21) is provided for placement inside said tank or box (12, 33), on or in which rack (21) at least one of said utensils (16) can be placed.

9. Steam cleaner (1) according to any one of the previous claims, wherein the tank or box (12, 33) is provided with a slot (26) in a wall part such as a wall or a lid, wherein said slot (26) is covered by a cover, the at least one inlet (18) being provided in or on said slot (26) for introducing steam between said slot (26) and said cover.

10. Steam cleaner (1) according to any one of the previous claims, wherein at least two connecting sockets (13) are provided.

11. Steam cleaner (1) according to any one of the previous claims, wherein the steam cleaner (1) comprises a housing (2), whereby the water tank (7) is provided on and/or in a sliding element (5), such that by sliding said sliding element (5) at least the water tank (7) can be brought out of said housing (2).

12. Steam cleaner (1) according to claim 11, wherein a control device (10) is provided for at least said heating means (8), which control device (10) is at least partly provided in or on said sliding element (5).

13. Steam cleaner (1) according to any one of the previous claims, wherein at least one receptacle (37) is provided for receiving cloth (38).

14. Steam cleaner (1) according to any one of the previous claims, wherein a connector is provided between said heating means (8) or steam generating means and said tank or box (12, 33)for introducing said steam into said tank or box (12, 33).

15. Method for cleaning, wherein at least one artefact is cleaned using the steam cleaner (1) of any preceding claim with at least one cleaning utensil (16), wherein after cleaning at least part of said artefact, said at least one cleaning utensil (16) is positioned in a tank or box (12, 33), wherein steam generated with said steam cleaner (1) is introduced into said tank or box (12, 33), for cleaning and preferably sterilising said artefact.

16. Method according to claim 15, wherein steam is introduced into said tank or box (12, 33) after hermetically closing it.

17. Method according to claim 15 or 16, wherein said utensils (16) are positioned in or on a rack (21) within said tank or box (12, 33), such that they are positioned above a bottom thereof.

18. Set of a steam cleaner (1) according to any one of claims 1 to 14 and a set of utensils (16) for said steam cleaner (1), wherein at least a number of said utensils (16) can be received with the tank or box (12, 33) of said steam cleaner (1).

## Patentansprüche

1. Dampfreiniger (1), der einen Wassertank (7), eine Heizeinrichtung (8) zur Dampfbildung und mindestens eine Verbindungsmuffe (13) für einen Dampfschlauch (14) umfasst, wobei ferner ein Tank oder ein Kasten (12, 33) vorgesehen ist, **dadurch gekennzeichnet, dass** der Tank oder Kasten mit mindestens einem Einlass (18) für Dampf in den genannten Tank oder Kasten (12, 33) vorgesehen ist, mit einem Aufnahmetank (12) für Abfall und mit einer Saugvorrichtung (11) zum Saugen von Abfall in den genannten Aufnahmetank (12).

2. Dampfreiniger (1) nach Anspruch 1, wobei der genannte Tank oder Kasten einen Werkzeugkasten (33) umfasst.

3. Dampfreiniger (1) nach Anspruch 1 oder 2, wobei der genannte Tank oder Katen (12, 33) mit einem Deckel (17) versehen ist, wobei der genannte mindestens eine Einlass (18) in oder in der Nähe des genannten Deckels (17) vorgesehen ist.

4. Dampfreiniger (1) nach einem der vorstehenden Ansprüche, wobei der Tank oder Kasten (12, 33) mit einer Reihe von Einlässen (18) versehen ist.

5. Dampfreiniger (1) nach einem der vorstehenden Ansprüche, wobei mindestens ein Dampfschlauch (14) vorgesehen ist, mit einem ersten Ende, das sich in Kooperation mit der mindestens einen Muffe (13) befindet, und mit einem entgegengesetzten Ende, das mit einer Kopplungseinrichtung zur Kopplung mit dem mindestens einen Einlass (18) vorgesehen ist.

6. Dampfreiniger (1) nach einem der vorstehenden Ansprüche, wobei der Tank (12) oder Kasten (33) mit einer Auslassöffnung (22) versehen ist, wobei diese vorzugsweise mit einem Verschluss (23) versehen ist.

7. Dampfreiniger (1) nach einem der vorstehenden Ansprüche, wobei Reinigungsvorrichtungen (16) zur Verbindung mit der genannten mindestens einen Muffe (13), vorzugweise über einen Dampfschlauch (14), vorgesehen sind, oder zum separaten Einsatz in Verbindung mit dem genannten Dampfreiniger (1), wobei mindestens eine der genannten Vorrichtungen (16) innerhalb des genannten Tanks oder Kastens (12, 33) platziert werden kann.

8. Dampfreiniger (1) nach Anspruch 7, wobei ein Gestell (21) zur Platzierung innerhalb des genannten Tanks oder Kastens (12, 33) vorgesehen ist, wobei auf oder in dem genannten Gestell (21) mindestens eine der genannten Vorrichtungen (16) platziert werden kann.

9. Dampfreiniger (1) nach einem der vorstehenden Ansprüche, wobei der genannte Tank oder Kasten (12, 33) mit einem Schlitz (26) in einem Wandteilstück wie etwa einer Wand oder einem Deckel versehen ist, wobei der genannte Schlitz (26) durch eine Abdeckung abgedeckt wird, wobei der mindestens eine Einlass (18) in oder an dem genannten Schlitz (26) vorgesehen ist, um Dampf zwischen dem genannten Schlitz (26) und der genannten Abdeckung einzuführen.

10. Dampfreiniger (1) nach einem der vorstehenden Ansprüche, wobei mindestens zwei Verbindungsmuffen (13) vorgesehen sind.

11. Dampfreiniger (1) nach einem der vorstehenden Ansprüche, wobei der Dampfreiniger (1) ein Gehäuse (2) umfasst, wobei der Wassertank (7) an und/oder in einem Schiebeelement (5) vorgesehen ist, so dass durch Verschieben des genannten Schiebeelements (5) zumindest der Wassertank (7) aus dem genannten Gehäuse (2) geführt werden kann.

12. Dampfreiniger (1) nach Anspruch 11, wobei eine Regelungsvorrichtung (10) zumindest für die genannte Heizeinrichtung (8) vorgesehen ist, wobei die Regelungsvorrichtung (10) zumindest teilweise in oder an dem genannten Schiebeelement (5) vorgesehen ist.

13. Dampfreiniger (1) nach einem der vorstehenden Ansprüche, wobei mindestens eine Aufnahmeeinrichtung (37) zur Aufnahme von Stoff (38) vorgesehen ist.

14. Dampfreiniger (1) nach einem der vorstehenden Ansprüche, wobei eine Verbindungseinrichtung zwischen der genannten Heizeinrichtung (8) oder der Dampferzeugungseinrichtung und dem genannten Tank oder Kasten (12, 33) vorgesehen ist, um den genannten Dampf in den genannten Tank oder Kasten (12, 33) einzuführen.

15. Verfahren zum Reinigen, wobei mindestens ein Gegenstand unter Verwendung des Dampfreinigers (1) nach einem der vorstehenden Ansprüche mit mindestens einer Reinigungsvorrichtung (16) gereinigt wird, wobei nach dem Reinigen zumindest eines Teils des genannten Gegenstands die genannte mindestens eine Reinigungsvorrichtung (16) in einem Tank oder Kasten (12, 33) positioniert wird, wobei mithilfe des genannten Dampfreinigers (1) erzeugter Dampf zum Reinigen und vorzugsweise zum Sterilisieren des genannten Gegenstands in den genannten Tank oder Kasten (12, 33) eingeführt wird.

16. Verfahren nach Anspruch 15, wobei Dampf in den genannten Tank oder Kasten (12, 33) eingeführt wird, nachdem dieser hermetisch verschlossen worden ist.

17. Verfahren nach Anspruch 15 oder 16, wobei die genannten Vorrichtungen (16) in oder auf einem Gestell (21) in dem genannten Tank oder Kasten (12, 33) positioniert werden, so dass sie oberhalb eines Bodens dessen positioniert werden.

18. Set eines Dampfreinigers (1) nach einem der Ansprüche 1 bis 14 und ein Set von Vorrichtungen (16) für den genannten Dampfreiniger (1), wobei zumindest eine Anzahl der genannten Vorrichtungen (16) in dem Tank oder dem Kasten (12, 33) des genannten Dampfreinigers (1) aufgenommen werden kann.

## Revendications

1. Appareil de nettoyage à la vapeur (1), comprenant un réservoir d'eau (7), des moyens de chauffage (8) pour former de la vapeur et au moins une tubulure de raccordement (13) pour un tuyau de vapeur (14), dans lequel, en outre, un réservoir ou une boîte (12, 33) est fourni, **caractérisé en ce que** le réservoir ou la boîte est pourvu au moins d'une entrée (18) pour la vapeur d'eau dans ledit réservoir ou caisse (12, 33), d'un réservoir de réception (12) pour les déchets et d'un dispositif d'aspiration (11) pour aspirer les déchets dans ledit réservoir de réception (12).

2. Appareil de nettoyage à la vapeur (1) selon la revendication 1, dans lequel ledit réservoir ou boîte comprend une boîte à outils (33).

3. Appareil de nettoyage à la vapeur (1) selon la revendication 1 ou 2, dans lequel ledit réservoir ou la boîte (12, 33) est pourvu d'un couvercle (17), dans lequel ladite au moins une entrée (18) est prévue dans ou à proximité dudit couvercle (17).

4. Appareil de nettoyage à la vapeur (1) selon l'une quelconque des revendications précédentes, dans lequel le réservoir ou la boîte (12, 33) est pourvu d'une série d'entrées (18).

5. Appareil de nettoyage à la vapeur (1) selon l'une quelconque des revendications précédentes, dans lequel au moins un tuyau de vapeur (14) est prévu, ayant une première extrémité pour coopérer avec l'au moins une tubulure (13) et une seconde extrémité opposée pourvue d'un coupleur pour le couplage à l'au moins une entrée (18).

6. Appareil de nettoyage à la vapeur (1) selon l'une quelconque des revendications précédentes, dans lequel le réservoir (12) ou la boîte (33) est pourvu d'une ouverture de sortie (22), de préférence munie d'une fermeture (23).

7. Appareil de nettoyage à la vapeur (1) selon l'une quelconque des revendications précédentes, dans lequel des ustensiles de nettoyage (16) sont prévus pour la connexion à ladite au moins une tubulure (13), de préférence par l'intermédiaire d'un tuyau de vapeur (14), ou pour une utilisation avec ledit dispositif de nettoyage à la vapeur (1) séparément, dans lequel au moins un desdits ustensiles (16) peut être placé à l'intérieur dudit réservoir ou caisse (12, 33).

8. Appareil de nettoyage à la vapeur (1) selon la revendication 7, dans lequel une crémaillère (21) est prévue pour être placée à l'intérieur dudit réservoir ou caisse (12, 33), sur ou dans laquelle crémaillère (21) au moins un desdits ustensiles (16) peut être placé.

9. Appareil de nettoyage à la vapeur (1) selon l'une quelconque des revendications précédentes, dans lequel le réservoir ou la boîte (12, 33) est pourvu d'une fente (26) dans une partie de paroi telle qu'un mur ou un couvercle, dans lequel ladite fente (26) est couverte par un couvercle, l'au moins une entrée (18) étant prévue dans ou sur ladite fente (26) pour introduire de la vapeur entre ladite fente (26) et ledit couvercle.

10. Appareil de nettoyage à la vapeur (1) selon l'une quelconque des revendications précédentes, dans lequel au moins deux tubulures de connexion (13) sont prévues.

11. Appareil de nettoyage à la vapeur (1) selon l'une quelconque des revendications précédentes, dans lequel l'appareil de nettoyage à la vapeur (1) comprend un boîtier (2), de sorte que le réservoir d'eau (7) est prévu sur et/ou dans un élément coulissant (5), de sorte qu'en coulissant ledit élément coulissant (5) au moins le réservoir d'eau (7) peut être amené hors dudit boîtier (2).

12. Appareil de nettoyage à la vapeur (1) selon la revendication 11, dans lequel un dispositif de commande (10) est prévu pour au moins lesdits moyens de chauffage (8), lequel dispositif de commande (10) est au moins partiellement prévu dans ou sur ledit élément coulissant (5).

13. Appareil de nettoyage à la vapeur (1) selon l'une quelconque des revendications précédentes, dans lequel au moins un réceptacle (37) est prévu pour recevoir un tissu (38).

14. Appareil de nettoyage à la vapeur (1) selon l'une quelconque des revendications précédentes, dans lequel un connecteur est prévu entre lesdits moyens de chauffage (8) ou moyens de production de vapeur et ledit réservoir ou caisse (12, 33) pour introduire ladite vapeur dans ledit réservoir ou boîte (12, 33).

15. Procédé de nettoyage, dans lequel au moins un objet est nettoyé à l'aide du jet de vapeur (1) selon l'une quelconque des revendications précédentes, avec au moins un ustensile de nettoyage (16), dans lequel, après le nettoyage d'au moins une partie dudit objet, ledit au moins un ustensile de nettoyage (16) est placé dans un réservoir ou caisse (12, 33), dans lequel la vapeur générée par ledit dispositif de nettoyage à la vapeur (1) est introduit dans ledit réservoir ou caisse (12, 33), pour le nettoyage et de préférence la stérilisation dudit objet.

16. Procédé selon la revendication 15, dans lequel la vapeur est introduite dans ledit réservoir ou caisse (12, 33) après sa fermeture hermétique.

17. Procédé selon la revendication 15 ou 16, dans lequel lesdits ustensiles (16) sont positionnés dans ou sur un support (21) à l'intérieur dudit réservoir ou boîte (12, 33), de telle sorte qu'ils sont positionnés au-dessus d'une partie inférieure de celui-ci.

18. Ensemble d'appareil de nettoyage à la vapeur (1) selon l'une quelconque des revendications 1 à 14 et ensemble d'ustensiles (16) pour ledit dispositif de nettoyage à la vapeur (1), dans lequel au moins un certain nombre desdits ustensiles (16) peut être reçu avec le réservoir ou la boîte (12, 33) dudit appareil de nettoyage à vapeur (1).
